(19) Europäisches Patentamt European Patent Office Office européen des brevets

(11) **EP 3 363 367 B1**

(12) **EUROPEAN PATENT SPECIFICATION**

(45) Date of publication and mention
of the grant of the patent:
**19.08.2020 Bulletin 2020/34**

(51) Int Cl.:
***A61B 8/08*** *(2006.01)*   ***A61B 8/00*** *(2006.01)*

(21) Application number: **17202032.3**

(22) Date of filing: **16.11.2017**

(54) **BODY TISSUE LOCATION MEASUREMENT SYSTEM**

KÖRPERGEWEBELOKALISIERUNGSMESSSYSTEM

SYSTÈME DE MESURE DE LOCALISATION DE TISSU CORPOREL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.02.2017 JP 2017028597**

(43) Date of publication of application:
**22.08.2018 Bulletin 2018/34**

(73) Proprietor: **Hitachi, Ltd.**
**Tokyo 100-8280 (JP)**

(72) Inventors:
• **KITAOKA, Masanori**
**Tokyo, 100-8280 (JP)**
• **KOURAI, Yuusuke**
**Tokyo, 100-8280 (JP)**
• **NAKANO, Hiroyuki**
**Tokyo, 100-8280 (JP)**

(74) Representative: **MERH-IP Matias Erny Reichl
Hoffmann**
**Patentanwälte PartG mbB**
**Paul-Heyse-Strasse 29**
**80336 München (DE)**

(56) References cited:
WO-A1-2016/209398    JP-A- H0 884 740
US-A1- 2013 150 756    US-A1- 2015 051 480

## Description

Technical Field

The present invention relates to a measurement system for measuring the location of a particular tissue in the body by means of ultrasonic waves.

Background Art

There are mainly three types of cancer treatment: surgery, chemotherapy, and radiation therapy. Radiation therapy is a method of treatment by irradiating a body tissue, which is the treatment target part of a patient, with a high dose of radiation. Radiation therapy allows the radiation to be concentrated on the part to be treated and thus has relatively few side effects. In order to perform radiation therapy effectively, it is important that the irradiation region to which radiation is concentrated and the region in which cancer to be treated is present match with high accuracy.

One of the methods for measuring the location of body tissue is described in Patent Literature 1.

Citation List

Patent Literature

Patent Literature 1: Japanese Unexamined Patent Application Publication No. 2003-117010

Patent literature US 2015/051480 A1 describes a method for tracing a movement trajectory of a lesion in a patient's moving internal structure by using a fixed therapy irradiation device that allows for a focal point on which an ultrasound wave emitted from elements of the irradiation device converges to change even though the therapy device is fixedly installed.

Summary of Invention

Technical Problem

The conventional radiation therapy system is designed to treat a patient by identifying the location of a body tissue, which is the part to be treated, from information such as previously obtained CT (Computed Tomography) images, creating a treatment plan based on the identification result, fixing the patient to the treatment table of the radiation therapy system, and irradiating the body tissue, which is the treatment target part of the patient, while controlling the characteristics of radiation such as irradiation direction and intensity.

However, performing an accurate treatment has been a problem, because the treatment target part of the patient moves from the previously planned radiation irradiation point due to the respiration of the patient during radiation therapy or other reasons . In order to solve this problem, a method for performing an accurate treatment has been established, in which a maker of gold or the like is implanted into the patient's body in advance to be used for previous treatment planning and radiation control during irradiation by photographing the marker in an X-ray transmission image and tracing the image to detect the movement of the treatment target part.

Further, in a system such as a proton therapy system in which positioning is performed by fixing the patient, there may be a case in which a CT image is obtained within a rotating gantry of the proton therapy system to confirm that the patient was finally fixed at the position consistent with the treatment plan. In such an additional CT, for example, a device such as a cone-beam CT is used. However, the photographing of the CT image may not be completed during the patient's breath hold due to the limitations of the rotating gantry. In this case, the body tissue, which is the treatment target part of the patient, moves due to the respiration of the patient or other reasons. As a result, the X-ray signal is integrated and the image quality is degraded.

Thus, it is desired to provide a low-invasive body tissue location measurement system that can reduce the need for marker implantation and the X-ray exposure dose during the treatment while being able to cope with respiration. An example of the low-invasive method is a method using ultrasonic images.

Patent Literature 1 describes a radiation therapy system that delivers radiation at the timing in which the correlation value between the ultrasonic image obtained in the same time phase as the previously obtained CT image and the ultrasonic image obtained during treatment is high, instead of using the marker and the X-ray.

The radiation therapy system described in Patent Literature 1 is designed to be able to achieve a low invasive treatment by controlling radiation to be delivered in accordance with a high correlation value between the ultrasonic image for treatment planning obtained in the same time phase as the previously obtained CT image and the ultrasonic image for treatment obtained during treatment. However, there is a problem with this radiation therapy system in that an ultrasonic probe attached to the end of a robot arm is pressed against and fixed to the patient's skin, which may cause the generation of ultrasonic elements that interfere with bone or radiation passing area depending on the part to be treated, so that a wide angle and clear ultrasonic image may not be obtained.

Further, there is also a problem that the way of pressing the organ tissue of the patient by the ultrasonic probe varies when the pressing of the ultrasonic probe is not controlled precisely and consistently from the treatment planning stage to the actual treatment, and as a result, the ultrasonic image for treatment to obtain the correlation value is changed due to respiration.

Furthermore, the above system is designed to deliver radiation in accordance with the acquisition of the ultrasonic image for treatment planning that presents a high correlation value with the treatment ultrasonic image, in which the location of the body tissue under treatment is not identified from the ultrasonic image. Thus, there is a risk that a positional error may occur with respect to the actual treatment target part.

The present invention has been made in view of the above problems, and an object of the present invention is to provide a body tissue location measurement system that can obtain a wide angle and clear ultrasonic image without distortion due to respiration through the effective use of ultrasonic element, in order to measure the location of a body tissue in a minimally invasive manner with high accuracy.

Solution to Problem

In order to solve the above described problems, for example, the configuration described in the claims is employed.

The present invention includes a plurality of means for solving the above described problems. To cite an example, one includes: a plurality of ultrasonic elements placed in different parts of a body; an external sensor for measuring a position of the ultrasonic elements; an ultrasonic propagation model holding unit for holding an ultrasonic propagation model; an ultrasonic propagation time calculation unit for calculating an ultrasonic propagation time based on the ultrasonic model and the ultrasonic element location; an ultrasonic image configuration unit for configuring an ultrasonic image from the ultrasonic propagation time as well as an ultrasonic signal received by each element; and/or a body tissue location calculation unit for calculating a location of a target body tissue from the ultrasonic image.

Advantageous Effects of Invention

According to the present invention, it is possible to obtain a wide angle and clear ultrasonic image without distortion due to respiration through the effective use of ultrasonic element, making it possible to measure the location of a body tissue in a minimally invasive manner with high accuracy. Brief Description of Drawings

Fig. 1 is a conceptual diagram of a body tissue location measurement system according to a first embodiment of the present invention;
Fig. 2 is a schematic view showing a cross section of a patient in a certain respiratory phase in the body tissue location measurement system of Fig. 1;
Fig. 3 is a schematic view showing the cross section of the same patient in another respiratory phase different, which is from Fig. 2, in the body tissue location measurement system of Fig. 1;
Fig. 4 is a conceptual diagram of the body tissue location measurement system in the generation of an ultrasonic propagation model according to the first embodiment of the present invention;
Fig. 5 is a flow chart of the generation of an ultrasonic propagation model according to the first embodiment of the present invention;
Fig. 6 is a schematic view showing an ultrasonic propagation signal according to the first embodiment of the present invention;
Fig. 7 is a conceptual diagram of a database of ultrasonic propagation models according to the first embodiment of the present invention;
Fig. 8 is a flow chart showing an ultrasonic image configuration method according to the first embodiment of the present invention;
Fig. 9 is a flow chart showing a body tissue location calculation method according to the first embodiment of the present invention;
Fig. 10 is a conceptual diagram showing a method of applying a body tissue location calculation device to a radiation delivery system according to a second embodiment of the present invention;
Fig. 11 is a conceptual diagram showing a method of applying the body tissue location calculation device to the radiation delivery system according to a third embodiment of the present invention; and
Fig. 12 is a conceptual diagram showing the body tissue location calculation device according to a fourth embodiment of the present invention.

Description of Embodiments

Hereinafter, embodiments will be described with reference to the accompanying drawings.

First Embodiment

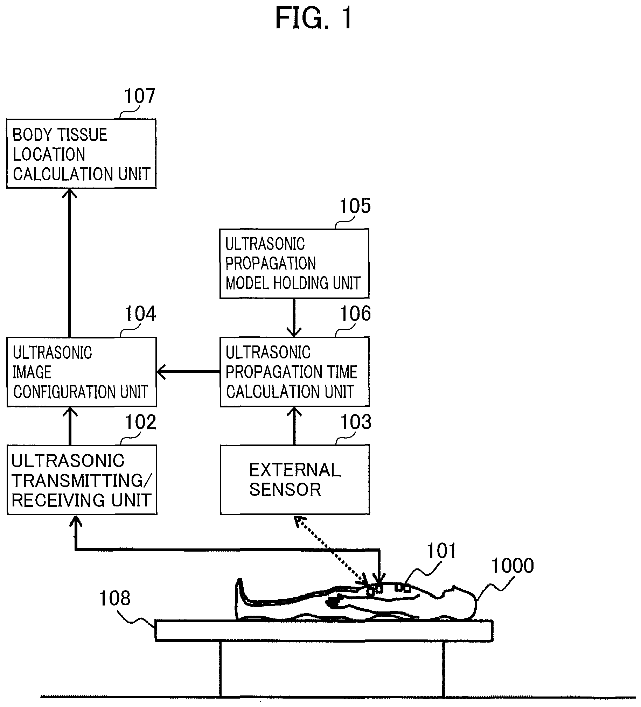

Hereinafter, a body tissue location measurement system according to a first embodiment of the present invention will be described with reference to Figs. 1 to 9.

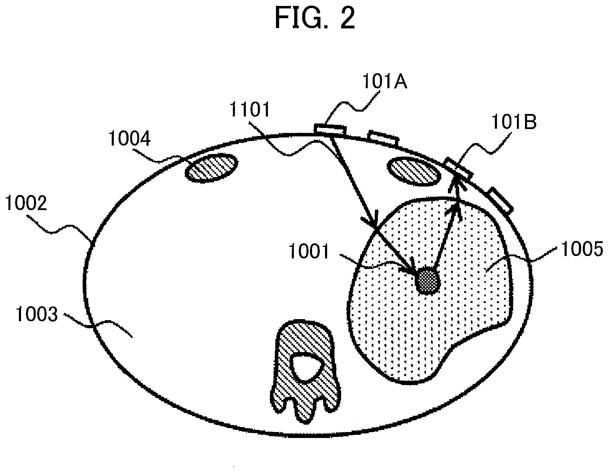

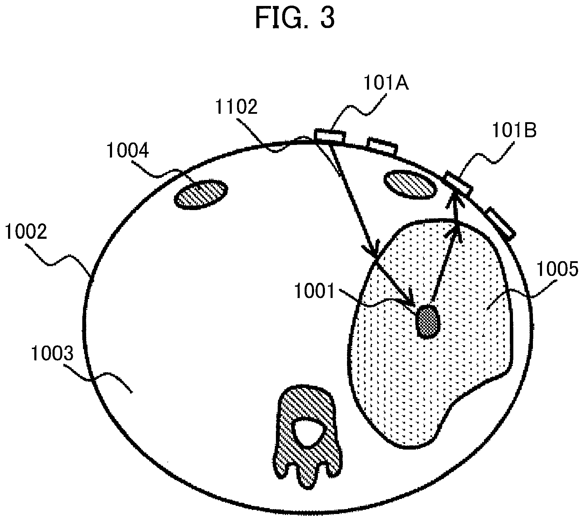

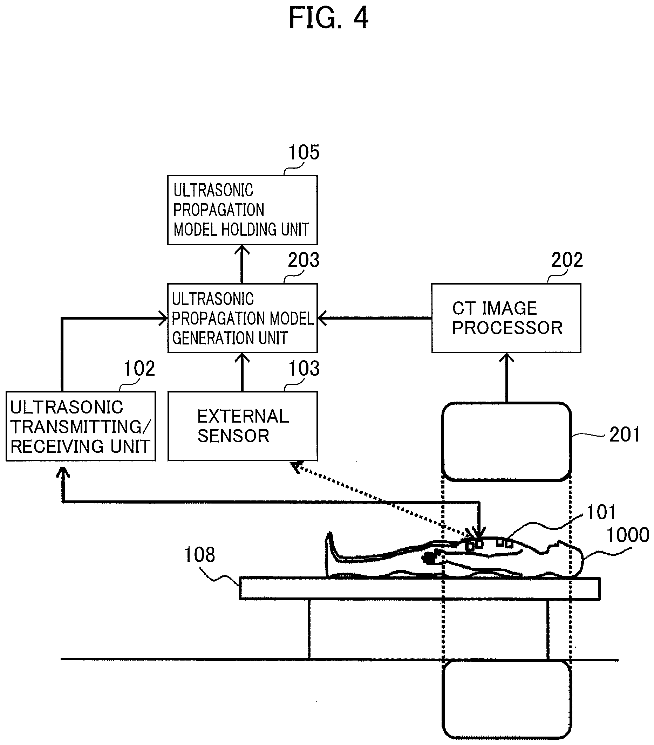

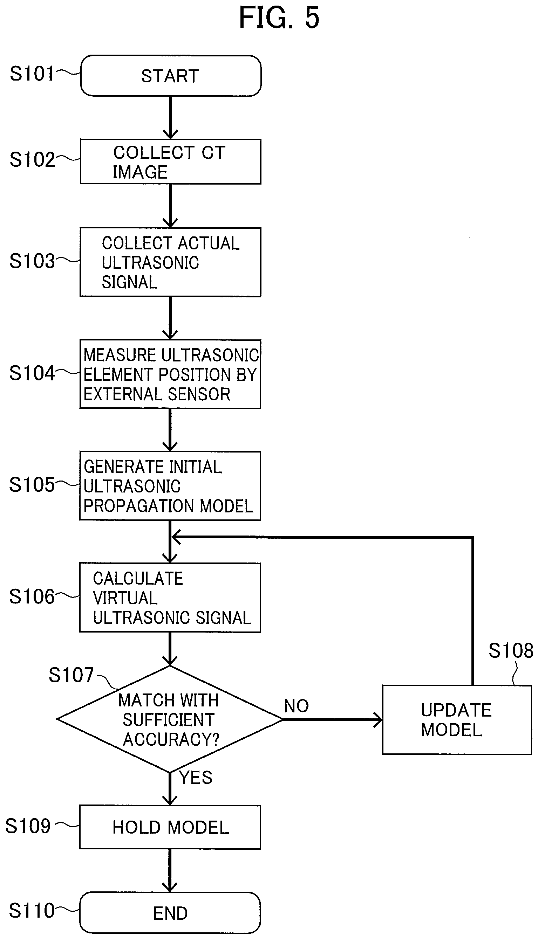

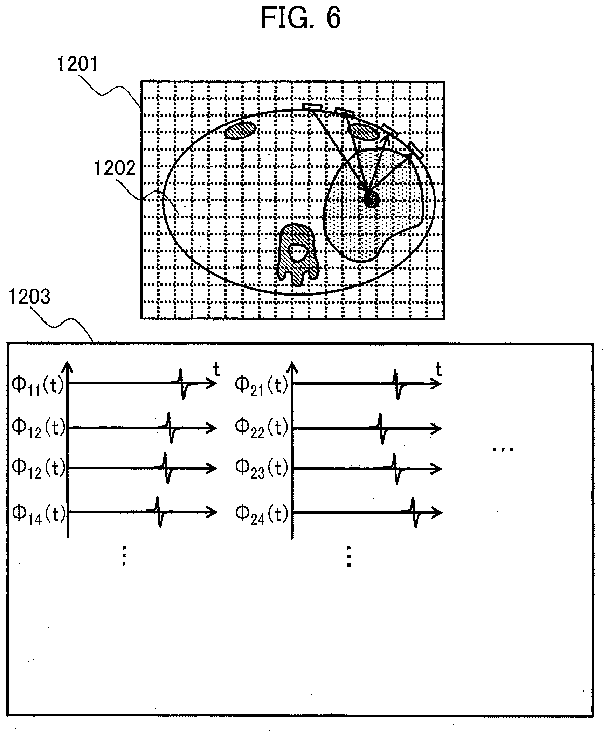

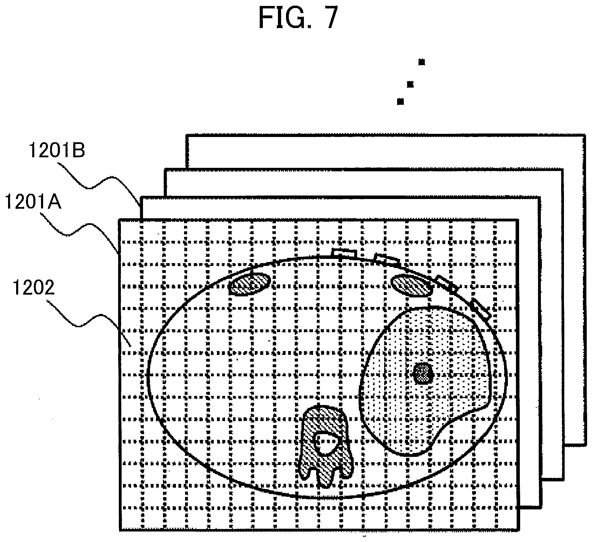

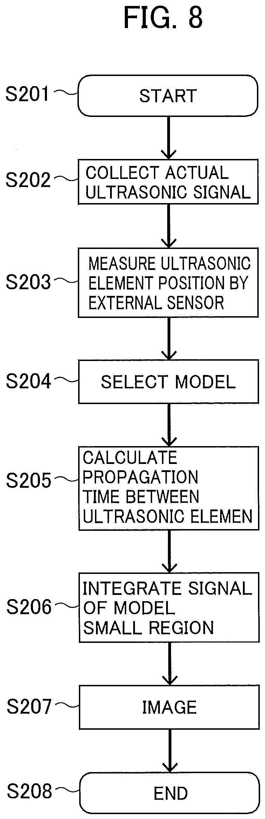

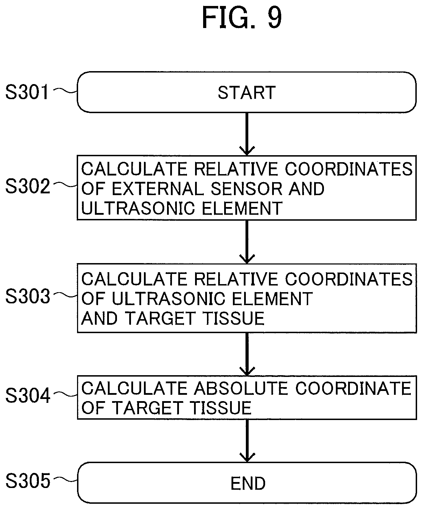

Fig. 1 is a conceptual diagram of the body tissue location measurement system according to the present embodiment. Fig. 2 is a schematic view showing a cross section of a patient in a certain respiratory phase. Fig. 3 is a schematic view showing the cross section of the same patient in another respiratory phase that is different from Fig. 2. Fig. 4 is a conceptual diagram of the body tissue location measurement system in the generation of the ultrasonic model according to the present embodiment. Fig. 5 is a flow chart of ultrasonic propagation model generation. Fig. 6 is a schematic view showing an ultrasonic propagation signal obtained in the present embodiment. Fig. 7 is a conceptual diagram of ultrasonic propagation model. Fig. 8 is a flow chart showing ultrasonic image configuration method. Fig. 9 is a flow chart showing body tissue location calculation method. Note that the parts common to those shown in Figs. 1 to 9 are denoted by the same reference numerals.

First, the configuration and function of the body tissue location measurement system according to the present embodiment will be described with reference to Figs. 1 to 4.

In Fig. 1, the body tissue location measurement system is to measure the location of an arbitrary body tissue within the body of a patient 1000 lying on a bed 108 and is fixed there. The body tissue location measurement system includes: a plurality of ultrasonic elements 101 placed in different parts on the surface of the body, preferably, the elements being closely attached thereto; an ultrasonic transmitting/receiving unit 102; an external sensor 103; an ultrasonic image configuration unit 104; an ultrasonic propagation model holding unit 105; an ultrasonic propagation time calculation unit 106; and a body tissue location calculation unit 107.

The patient 1000 is fixed to the bed 108 in a state in which the ultrasonic elements 101 are closely attached to the body surface. The ultrasonic element receives an electrical signal from the ultrasonic transmitting/receiving unit 102, excites ultrasonic waves at an arbitrary timing, and transmits the ultrasonic waves to the body of the patient. Further, the ultrasonic element receives ultrasonic waves returning from the body of the patient due to reflection, scattering, or the like, converts the received ultrasonic waves into an electrical signal, and transmits to the ultrasonic transmitting/receiving unit 102. The ultrasonic transmitting/receiving unit 102 amplifies and processes the electrical signal received from the ultrasonic element 101, and transmits to the ultrasonic image configuration unit 104. A plurality of ultrasonic elements are used at the same time. However, unlike the ultrasonic probes generally used in ultrasonic diagnostic apparatus, the relative position among the ultrasonic elements is not required to be fixed. The ultrasonic elements are provided independently so that their relative position varies in accordance with movement such as the respiration of the patient 1000.

The external sensor 103 is the device that measures the position of the ultrasonic element 101. The external sensor 103 measures the absolute coordinates with respect to an appropriate absolute coordinate origin, such as a particular point of the external sensor 103 itself or the bed 108.

Here, for example, a laser distance meter can be used as the external sensor 103. In this case, it is possible to identify the ultrasonic element position and movement, in such a way that the light is not absorbed into the ultrasonic element 101 by using a laser reflective material in the back surface of the ultrasonic element 101 (the side opposite to the side closely attached to the body surface 1002). Further, for example, one or a plurality of video cameras can be used as the external sensor 103. In this case, a pattern unique to each element is printed on the back surface of the ultrasonic element 101, so that the ultrasonic element position and movement can be identified, for example, by using a technique generally called image correlation method. Further, it may also be possible to measure the ultrasonic element position by using a contact sensor, a robot arm, or the like, as the external sensor 103. In this way, it is possible to use various methods for the external sensor 103. Thus, the example shown here is not intended to limit the embodiments of the present invention.

The ultrasonic propagation model holding unit 105 holds a previously generated ultrasonic model including the ultrasonic element 101 and the body tissue of the patient. The ultrasonic propagation model holding unit 105 can hold a plurality of ultrasonic models, select one of them and transmit to the ultrasonic propagation time calculation unit. The ultrasonic propagation time calculation unit 106 calculates the propagation time from when an ultrasonic wave transmitted from one of the ultrasonic elements is reflected at an arbitrary point within the ultrasonic propagation model until when the ultrasonic wave is received by another ultrasonic element, by using the ultrasonic propagation model held by the ultrasonic propagation holding unit 105.

The ultrasonic image configuration unit 104 configures an ultrasonic image by using the ultrasonic single transmitted from the ultrasonic transmitting/receiving unit 102 as well as the ultrasonic propagation time calculated by the ultrasonic propagation time calculation unit 106. The body tissue location calculation unit 107 calculates the absolute coordinates of the body tissue location by using the ultrasonic image configured by the ultrasonic image configuration unit 104 as well as the ultrasonic element position measured by the external sensor 103.

Figs. 2 and 3 are schematic views respectively showing a cross section of a patient in a certain respiratory phase. Figs. 2 and 3 show cross sections of respective respiratory phases of one patient. The interior of the patient's body includes body surface 1002, muscle and fat 1003, bone 1004, organ 105, and the like, in addition to a body tissue 1001 which is the target for location measurement. Ultrasonic elements 101A and 101B are provided to be able to access the body tissue 1001, while avoiding obstacles that block ultrasonic waves, such as the bone 1004.

An ultrasonic propagation path 1101 is an example of a propagation path in which the ultrasonic wave transmitted from the ultrasonic element 101A, which is one of the ultrasonic elements, is reflected at an arbitrary point within the body and received by another ultrasonic element 101B. As is clear from the example, the ultrasonic propagation path 1101 changes according to the state of the patient 1000 because the relative position with respect to the bone 1004, the organ 1005, and the like changes in accordance with movement such as the respiration of the patient 1000. Further, for example, when the ultrasonic wave propagates from the fat to the organ, the so-called acoustic impedance changes due to the differences in density and acoustic velocity, so that the ultrasonic wave travels while being refracted. Here, it is known that the acoustic velocity within the fat is about 1450 m/s, the acoustic velocity within the blood, muscle, and organ is about 1530 to 1630 m/s, and the acoustic velocity within the bone is about 2700 to 4100 m/s.

Fig. 4 is a conceptual diagram of the body tissue location measurement system in the ultrasonic propagation model generation. In the ultrasonic propagation model generation, a CT gantry 201, a CT image processor 202, and an ultrasonic propagation model generation unit 203 are added to the configuration in Fig. 1. The parts common to those shown in Fig. 1 are denoted by the same reference numerals . Further, the parts not required for the ultrasonic propagation model generation are omitted here.

The CT gantry 201 and the CT image processor 202 obtain a CT image of a region including at least the ultrasonic element 101 of the patient 1000 as well as the body tissue 1001 which is the target for location measurement. The obtained CT image is transmitted to the ultrasonic propagation model generation unit 203. At this time, for example, the movement of the body surface 1002 is monitored using the measurement result of the external sensor to obtain respiratory gated CT images in order to collect a plurality of CT images.

The ultrasonic propagation model generation unit 203 generates an ultrasonic propagation model by combining the ultrasonic signal received by the ultrasonic transmitting/receiving unit 102, the position of the ultrasonic element 101 measured by the external sensor 103, and the CT images collected by the CT image processor. Then, the generated model is held by the ultrasonic propagation model holding unit 105.

Here, the ultrasonic transmitting/receiving unit 102, the ultrasonic image configuration unit 104, the ultrasonic propagation time calculation unit 106, the body tissue location calculation unit 107, and the ultrasonic propagation model generation unit 203 perform their calculations by the program loaded into a computer, FPGA, or other device. The ultrasonic propagation model holding unit can be configured with a memory, an external storage device, a database, and the like.

Next, the ultrasonic propagation model generation method in the body tissue location measurement system according to the present invention will be described with reference to Figs. 5 to 7.

Fig. 5 is a flow chart of the ultrasonic propagation model generation, and Fig. 6 is a conceptual diagram of the ultrasonic propagation model. The ultrasonic propagation model generation includes the steps shown in Fig. 5.

First, the ultrasonic propagation model generation method starts in step S101. Here, it is assumed that the patient 1000 has already been fixed to the bed 108, and devices such as the ultrasonic element 101, the external sensor 103, and the CT gantry 201 have been prepared.

In step S102, the method collects CT images in each respiratory phase of the patient 1000.

Next, in step S103, the method collects actual ultrasonic signals for each respiratory phase of the patient 1000. As shown in Fig. 6, an actual ultrasonic signal 1203 excites the provided ultrasonic elements 101 in series, and recodes all or part of the reception signal of the transmitted ultrasonic wave received by each of the ultrasonic elements. For example, when an ultrasonic wave is transmitted from the i-th ultrasonic element and is received by the j-th ultrasonic element, the reception signal is expressed as a function of time t by $\phi_{ij}(t)$ ($1 \leq i \leq N$, $1 \leq j \leq N$, $0 \leq t \leq T$). Here, N is the total number of provided ultrasonic elements and T is the collection time. It is assumed that the recording time T is sufficient time for collecting signals of the ultrasonic wave.

Next, in step S104, the method measures the ultrasonic element position by using the external sensor 103.

Here, steps S102 to S104 may be performed at the same time, and the order of the execution may be changed. In the collection of actual ultrasonic signals and in the collection of CT images, when it is difficult to perform step S102 and step S103 at the same time due to reasons such as that an artifact of the ultrasonic element appears in the CT image, it is preferable to collect data in synchronization with the patient's movement, such as respiration, by performing at least step S102 and step S104, and step S103 and step S104, respectively, at the same time and by recording the respiratory phase.

Next, in step S105, the method generates an initial ultrasonic propagation model. First, the method prepares a model 1201 within the patient's body, which is divided into appropriate small regions 1202 (meshes) in accordance with the CT image. It is possible to set a combination of physical quantities required for the calculation of the ultrasonic propagation, such as the density and acoustic velocity values or the acoustic impedance, in each small region. As for the initial ultrasonic propagation model, the step first discriminates body tissues, such as fat, muscle, blood vessel, bone, and organ, in accordance with the intensity of the CT image. Then, the method sets the physical quantity in each small region according to the type of the tissue.

Next, in step S106, the method calculates a virtual ultrasonic signal based on the initial ultrasonic propagation model as well as the ultrasonic element position obtained in S103, and gives the following expression: $\phi_{ij}(t)$ ($1 \leq i \leq N$, $1 \leq j \leq N$, $0 \leq t \leq T$).

In step S107, the method compares the actual ultrasonic signal $\phi_{ij}(t)$ with the virtual ultrasonic signal $\phi_{ij}(t)$ and determines whether the two signals match with sufficient accuracy. If it is determined that the two signals do not match, the method updates the ultrasonic propagation model in step S108, and returns to step S106. Updating the ultrasonic propagation model can be done, for example, by using a general optimization method.

If it is determined that the actual ultrasonic signal $\phi_{ij}(t)$ and the virtual ultrasonic signal $\phi_{ij}(t)$ match with sufficient accuracy in step S107, the method proceeds to step S109 and stores the ultrasonic propagation model in the ultrasonic propagation model holding unit 105.

Finally, the method proceeds to step S110 and ends the ultrasonic propagation model generation.

Fig. 7 is a conceptual diagram of the database of ultrasonic propagation models according to the present embodiment. When a plurality of ultrasonic propagation models are required in accordance with the movement associated with the respiration of the patient 1000, the method generates and stores ultrasonic propagation models by repeating the process from S105 to S109 as many times as the required number of ultrasonic propagation models. As a result, a plurality of ultrasonic propagation models such as ultrasonic propagation models 1201A, 1201B are stored in the ultrasonic propagation model holding unit 105.

By the steps described above, it is possible to generate the ultrasonic propagation models synchronized with the respiration, in such a way that the ultrasonic signal corresponds to the CT image.

Next, the ultrasonic image configuration method according to the present embodiment will be described with reference to Fig. 8.

Fig. 8 is a flow chart showing an ultrasonic image configuration method according to the present embodiment.

First, the ultrasonic image configuration method starts in step S201. Here, it is assumed that the patient 1000 has already been fixed to the bed 108, and the devices such as the ultrasonic elements 101 and the external sensor 103 have been prepared. Since step S202 and step S203 are respectively the same as the above described step S103 and step S104, the description thereof will be omitted.

In step S204, the method selects an ultrasonic propagation model to be used in the propagation time calculation unit 106. When the number of ultrasonic propagation models held in the ultrasonic propagation model holding unit 105 is only one, the method uses the particular ultrasonic propagation model. When a plurality of ultrasonic propagation models are held in the ultrasonic propagation model holding unit 105, the method selects an appropriate ultrasonic propagation model.

For example, the selection of the ultrasonic propagation model is done by identifying the respiratory phase of the patient 1000 based on the change in the ultrasonic element position obtained by the external sensor 103, and then selecting the ultrasonic propagation model with the same respiratory phase. Further, for example, the selection is done by comparing the ultrasonic signal collected in the ultrasonic propagation model generation with the ultrasonic signal obtained in step S202, and then selecting the ultrasonic propagation model corresponding to the case in which their correlation values are closest to each other. Furthermore, for example, the selection can also be done by performing a back-propagation analysis on the ultrasonic signal reflected by one part of the patient or by a specific one of a plurality of parts of the patient, from the ultrasonic signal obtained in step S202, and then selecting the ultrasonic propagation model with which the strongest signal is generated. In this way, various methods can be used for the selection of the ultrasonic propagation model. Thus, the example shown here is not intended to limit embodiments of the present invention.

Next, in step S205, by using the ultrasonic propagation model selected in step S204, the method calculates the propagation time $t_{ijk}$ when the ultrasonic signal that is transmitted from the i-th ultrasonic element and received by the j-th ultrasonic element, while being reflected within the k-th small region. In this calculation, for example, it is possible to use an analysis using the finite element method and ray tracing.

Here, the ultrasonic propagation time calculation requires calculation time. In order to reduce the calculation time, it may be possible to transmit an ultrasonic signal in advance by the i-th ultrasonic element in the ultrasonic model generation, calculate the propagation time $t_{ijk}$ in advance when the ultrasonic signal is received by the j-th ultrasonic element while being reflected within the k-th small region, and hold the propagation time $t_{ijk}$ in advance as one of the physical quantities of the respective small regions, and then read the data from the ultrasonic propagation model to obtain the propagation time $t_{ijk}$ required for the ultrasonic image configuration.

Next, in step S206, the method calculates the signal strength $I_k$ of the k-th small region by using the following equation 1.

$$\text{Equation 1}$$

$$I_k = \left| \sum_{i=1}^{N} \sum_{j=1}^{N} \phi_{ij}\left(t_{ijk}\right) \right|$$

Next, in step S207, the method calculates the intensity of each small region based on the signal strength $I_k$ and configures an ultrasonic image. Here, it may be possible to use the signal strength $I_k$ itself as the pixel intensity, or apply image processing to the ultrasonic image configuration obtained as described above so as to improve the image quality of the body tissue which is the target of position measurement.

Finally, the method proceeds to step S208 and ends the ultrasonic image configuration.

Note that while the present embodiment has descried the example of using a plurality of ultrasonic propagation models, it may also be possible to use only a model in a state in which the patient holds their breath, as long as sufficient accuracy is available. Further, in the ultrasonic model generation, the density and acoustic velocity may be set using information of only the CT image, instead of using the actual ultrasonic signal. In addition, it may be possible to simply model the human body by considering it as an object with uniform density and acoustic velocity, as long as sufficient accuracy is available.

Next, the body tissue location calculation method in the body tissue location calculation unit 107 will be described with reference to Fig. 9.

Fig. 9 is a flow chart showing a body tissue position calculation method according to the present invention. The body tissue location calculation method is configured with the following steps.

First, the body tissue location calculation method starts in step S301. Next, the method calculates the relative coordinates of the external sensor and the ultrasonic element in step S302. Then, in step S303, the method performs image processing using the ultrasonic image configured by the method described above, and calculates the relative coordinates of the ultrasonic element and the target body tissue. Finally, in step S304, the method calculates the absolute coordinates of the target body tissue by combining the relative coordinates calculated in step S302 and the relative coordinates calculated in step S303. In S305, the method transmits the calculation result to the subsequent device in which the calculation result is used, and ends the process.

In this way, a wide angle and clear ultrasonic image can be obtained without distortion due to respiration through the effective use of the ultrasonic element. This makes is possible to measure the body tissue location in a minimally invasive manner with high accuracy.

The body tissue location calculation device shown in the present embodiment may also be used as an ultrasonic diagnostic imaging system, by displaying the ultrasonic image in an ultrasonic image display device added to the ultrasonic image configuration unit 104. At this time, in order to allow the doctor to make an intuitive diagnosis, it may also be possible to display image data to which the image processing is applied in the same manner as in the conventional ultrasonic diagnostic imaging system, in addition to the pixel information given by the equation 1.

Second Embodiment

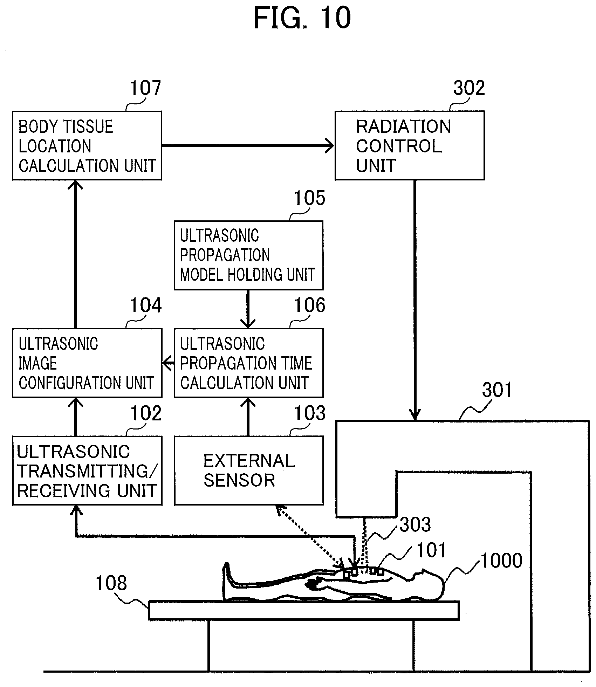

Next, a body tissue location calculation device according to a second embodiment not forming part of the invention will be described with reference to Fig. 10. Note that the parts in Fig. 10 common to those shown in Fig. 1 are denoted by the same reference numerals, and the description of the parts already described will be omitted.

Fig. 10 is a conceptual diagram showing a method of applying the body tissue location calculation device to the radiation delivery system according to the second embodiment. In addition to the first embodiment, the second embodiment further includes a radiation irradiation unit 301 and a radiation control unit 302. The radiation control unit 302 receives the body tissue location calculated by the body tissue location calculation unit 107 as a signal, and controls the irradiation point of radiation 303 such as X rays and particle beams with which the patient 1000 is irradiated, by controlling the radiation irradiation unit 301. In this way, it is possible to control the radiation irradiation point to concentrate the radiation on the region of the treatment target part that is planned in the treatment planning. Although the CT gantry 202, the ultrasonic propagation model generation unit 203, and the like are not shown in Fig. 10, the configuration involved in the model generation described with respect to Fig. 4 as well as a CT imaging system may also be provided in addition to the configuration described in Fig. 10. Note that the ultrasonic propagation model used for moving body tracing to trace the affected part is generated in advance, upon generation of the treatment plan for radiation therapy or just before the treatment.

At this time, by monitoring the respiratory state of the patient, it is also possible to start and stop delivery of radiation by identifying the respiratory phase and selecting the ultrasonic propagation model 1201, to configure an ultrasonic image in order to identify the appropriate timing when the treatment target part passes the intended coordinate region. Further, it is possible to control the radiation 303 according to the movement of the treatment target part by repeatedly performing the body tissue location calculation at an appropriate frame rate.

In this way, it is possible to obtain a wide angle and clear ultrasonic image without distortion due to respiration through the effective use of the ultrasonic element. This makes it possible to measure the location of a body tissue in a minimally invasive manner with high accuracy and to deliver the radiation to the treatment target part of the patient by precisely controlling the radiation irradiation point.

Third Embodiment

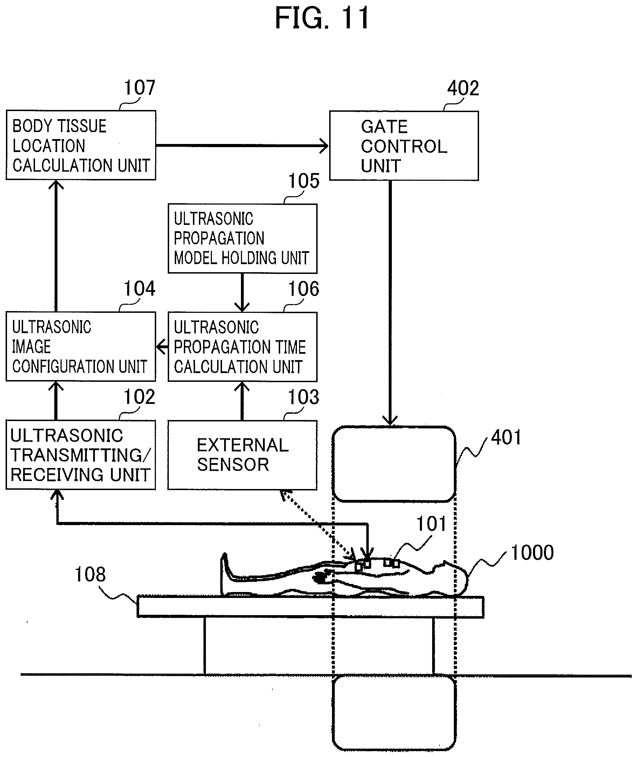

Next, a body tissue location calculation device according to a third embodiment of the present invention not forming part of the invention will be described with reference to Fig. 11. Note that the parts in Fig. 11 common to those shown in Fig. 1 are denoted by the same reference numerals, and the description of the parts already described will be omitted. Fig. 11 is a conceptual diagram showing a method of applying the body tissue location calculation device to the diagnostic imaging system according to the third embodiment. In addition to the first embodiment, the third embodiment further includes a diagnostic imaging system gantry 401 and a gate control unit 402. The gate control unit 402 receives the body tissue location calculated by the body tissue location calculation unit 107 as a signal, and controls the timing to photograph the patient 1000 by controlling the diagnostic imaging system gantry 401.

At this time, by monitoring the respiratory state of the patient, it is possible to perform photographing by identifying the respiratory phase and selecting the ultrasonic propagation model 1201 to configure an ultrasonic image in order to identify the appropriate timing when the treatment target part passes the intended coordinate region. Further, it is possible to perform photographing in accordance with the movement of the part to be photographed, by repeatedly performing the body tissue location calculation at an appropriate frame rate. In this way, it is possible to prevent blurring caused by the movement of the tumor at the time of photographing with the diagnostic imaging system and to obtain a clear image.

Fourth Embodiment

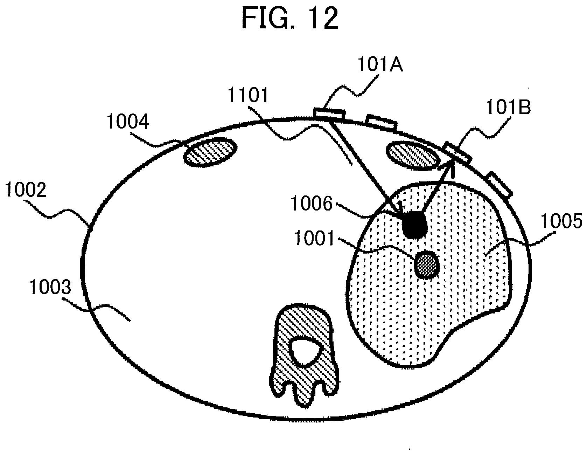

Next, a body tissue location calculation device according to a fourth embodiment not forming part of the invention will be described with reference to Fig. 12. Note that the parts in Fig. 12 common to those shown in Fig. 2 are denoted by the same reference numerals, and the description of the parts already described will be omitted.

Fig. 12 is a conceptual diagram of the body tissue location calculation device according to the fourth embodiment The present embodiment is configured such that an ultrasonic reflector 1006 that strongly reflects ultrasonic waves is implanted into the body of the patient 1000 to calculate the position of the ultrasonic reflector 1006.

It is generally known that biological tissues are soft tissues, in which the ultrasonic reflection is less likely to occur because the ultrasonic wave is greatly attenuated and the difference in the acoustic impedance between tissues in the body is small. Thus, it may be difficult to obtain sufficient reflected signal from the body tissue which is the target for location calculation. As a result, a clear ultrasonic image may not be obtained.

In the present embodiment, the ultrasonic reflector 1006, whose acoustic impedance is very different from the biological tissue, is implanted in the vicinity of the body tissue such as the particular treatment target part concerned in the body of the patient 1000 in advance before the ultrasonic model generation, in order to obtain a clearer image. At the stage of acquisition of CT image, if the relative coordinates of the position of the implanted ultrasonic reflector 1006 and the calculated location of the body tissue are calculated in advance, it is possible to calculate the location of the particular body tissue concerned by adding the previously calculated relative coordinates into the absolute coordinates of the ultrasonic reflector 1006.

As described above, it is possible to obtain a wide angle and clear ultrasonic image without distortion due to respiration through the effective use of the ultrasonic element. This makes it possible to measure the body tissue location while reducing the amount of X-ray exposure.

List of Reference Sings

101, 101A, 101B: ultrasonic element
102: ultrasonic transmitting/receiving unit
103: external sensor
104: ultrasonic image configuration unit
105: ultrasonic propagation model holding unit
106: ultrasonic propagation time calculation unit
107: body tissue location calculation unit
108: bed
201: CT gantry
202: CT image processor
203: ultrasonic propagation model generation unit
301: radiation irradiation unit
302: radiation control unit
303: radiation
401: diagnostic imaging system gantry
402: gate control unit
1000: patient
1001: target body tissue
1002: body surface
1003: fat and muscle
1004: bone
1005: organ
1006: ultrasonic reflector
1101, 1102: ultrasonic propagation path
1201, 1201A, 1201B: ultrasonic modelling region
1202: ultrasonic model small region
1203: ultrasonic signal

Claims

1. A body tissue location measurement system comprising:

a plurality of ultrasonic elements (101, 101A, 101B) placed in different parts on the surface of a body;
an external sensor (103) configured for measuring a position of the ultrasonic elements;
an ultrasonic propagation model holding unit (105) configured for holding an ultrasonic propagation model;
an ultrasonic propagation time calculation unit (106) configured for calculating an ultrasonic propagation time based on the ultrasonic model and the ultrasonic element position;
an ultrasonic image configuration unit (104) configured for configuring an ultrasonic image from the ultrasonic propagation time as well as an ultrasonic signal received by each element; and
a body tissue location calculation unit (107) configured for calculating a location of a target body tissue from the ultrasonic image,
an ultrasonic transmitting/receiving unit (102) configured for amplifying and processing electrical signals received from the ultrasonic elements (101), and for transmitting the electrical signals to the ultrasonic image configuration unit (104),
wherein
the ultrasonic elements (101) are provided independently, so that their relative position varies in accordance with movement.

**2.** The body tissue location measurement system according to claim 1, further comprising: a display device configured for making visible the ultrasonic image configured by the ultrasonic image configuration unit as well as the target body tissue location calculated by the body tissue location calculation unit.

**3.** The body tissue location measurement system according to claim 1, further comprising:

a CT image processor (202);
an ultrasonic model generation unit (203) configured for generating an ultrasonic model based on images collected by the CT image processor, the ultrasonic model, and the ultrasonic element position; and
holding means configured for storing the ultrasonic model in the ultrasonic propagation model holding unit.

**4.** The body tissue location measurement system according to claim 1, wherein:
an i-th ultrasonic element (101, 101A, 101B) in the ultrasonic model generation is configured:
to transmit an ultrasonic signal in advance, to calculate the propagation time in advance when the ultrasonic signal (1203) is received by a j-th ultrasonic element (101, 101A, 101B) while being reflected within a small region, to hold the propagation time in advance as one of the physical quantities of the respective small region, and then to read data from the ultrasonic propagation model to obtain the propagation time required for the ultrasonic image configuration.

**5.** A radiation therapy system comprising:

the body tissue location measurement system according to claim 1; and
a radiation control unit configured for controlling a radiation irradiation point based on a body tissue location measured by using the body tissue location measurement system.

**6.** A diagnostic imaging system comprising:

the body tissue location measurement system according to claim 1; and
a gate control unit (402) configured for controlling an imaging timing based on a body tissue location measured by using the body tissue location measurement system.

**7.** A body tissue location measurement method comprising:

a step of measuring a position of a plurality of ultrasonic elements (101, 101A, 101B), which are placed in different parts on the surface of a body by means of an external sensor (103);
a step of calculating a propagation path and time of an ultrasonic wave from an ultrasonic element to a position of another ultrasonic element by means of an ultrasonic propagation model;
a step of configuring an ultrasonic image from the ultrasonic propagation time and an ultrasonic signal received by each element;
a step of calculating a location of the target body tissue from the ultrasonic image; and
a step of amplifying and processing of electrical signals received from the ultrasonic elements (101) and transmitting the electrical signals to the ultrasonic image configuration unit (104) by means of an ultrasonic transmitting/receiving unit (102);
wherein
the ultrasonic elements are provided independently, so that their relative position varies in accordance with movement.

**8.** The body tissue location measurement method according to claim 7, further comprising a step of updating the calculation of an ultrasonic propagation model and a body tissue location in accordance with change in a contour of a patient.

**9.** The body tissue location measurement method according to claim 7, further comprising:

a step of generating an ultrasonic model; and
a step of storing in an ultrasonic model holding unit (105).

**10.** The body tissue location measurement method according to claim 9;
wherein the step of generating an ultrasonic model includes:

a step of collecting CT images for each respiratory phase;
a step of collecting actual ultrasonic signals in the same respiratory phase as the CT image by means of an ultrasonic element;
a step of measuring a position of the ultrasonic element in the same respiratory phase as the CT image by means of an external sensor (103);
a step of generating an initial model including an ultrasonic element and a treatment target part based on the CT image;
a step of dividing the initial model into small regions and setting a density and acoustic velocity in each small region;
a step of calculating a virtual ultrasonic signal based on the initial model, the ultrasonic element position, and the density and acoustic velocity; and

a step of comparing the actual ultrasonic signal (1203) with the virtual ultrasonic signal to update the density and acoustic velocity of the small region.

11. The body tissue location measurement method according to claim 10,
wherein the step of setting the density and acoustic velocity in the each small region sets the density and acoustic velocity based on the intensity of the CT image.

**Patentansprüche**

1. System zum Messen einer Körpergewebeposition, das Folgendes umfasst:

   mehrere Ultraschallelemente (101, 101A, 101B), die an verschiedenen Abschnitten auf der Oberfläche eines Körpers angeordnet werden;
   einen äußeren Sensor (103), der konfiguriert ist, eine Position der Ultraschallelemente zu messen;
   eine Einheit (105) zum Speichern eines Ultraschall-Ausbreitungsmodells, die konfiguriert ist, ein Ultraschall-Ausbreitungsmodell zu speichern;
   eine Einheit (106) zum Berechnen einer Ultraschall-Ausbreitungszeit, die konfiguriert ist, eine Ultraschall-Ausbreitungszeit auf der Basis des Ultraschallmodells und der Position des Ultraschallelements zu berechnen;
   eine Einheit (104) zum Konfigurieren eines Ultraschallbilds, die konfiguriert ist, ein Ultraschallbild aus der Ultraschall-Ausbreitungszeit sowie eines Ultraschallsignals, das durch das jeweilige Element empfangen wird, zu konfigurieren; und
   eine Einheit (107) zum Berechnen einer Körpergewebeposition, die konfiguriert ist, eine Position eines Ziel-Körpergewebes aus dem Ultraschallbild zu berechnen,
   eine Einheit (102) zum Senden und Empfangen von Ultraschall, die konfiguriert ist, elektrische Signale, die von den Ultraschallelementen (101) empfangen werden, zu verstärken und zu verarbeiten, und die elektrischen Signale an die Einheit (104) zum Konfigurieren des Ultraschallbilds zu senden,
   wobei die Ultraschallelemente (101) unabhängig voneinander vorgesehen sind, so dass sich ihre relative Position in Übereinstimmung mit einer Bewegung ändert.

2. System zum Messen einer Körpergewebeposition nach Anspruch 1, das ferner Folgendes umfasst:

   eine Anzeigevorrichtung, die konfiguriert ist, das Ultraschallbild, das durch die Einheit zum Konfigurieren des Ultraschallbilds konfiguriert wird, sowie die Ziel-Körpergewebeposition, die durch die Einheit zum Berechnen der Körpergewebeposition berechnet wird, sichtbar zu machen.

3. System zum Messen einer Körpergewebeposition nach Anspruch 1, das ferner Folgendes umfasst:

   einen CT-Bildprozessor (202);
   eine Einheit (203) zum Erzeugen eines Ultraschallmodells, die konfiguriert ist, auf der Basis von Bildern, die durch den CT-Bildprozessor gesammelt werden, des Ultraschallmodells und der Position des Ultraschallelements ein Ultraschallmodell zu erzeugen; und
   Speichermittel, die konfiguriert sind, das Ultraschallmodell in der Einheit zum Speichern des Ultraschall-Ausbreitungsmodells zu speichern.

4. System zum Messen einer Körpergewebeposition nach Anspruch 1, wobei:
ein i-tes Ultraschallelement (101, 101A, 101B) in der Ultraschallmodellerzeugung konfiguriert ist, die folgenden Schritte auszuführen:
Senden eines Ultraschallsignals im Voraus, um die Ausbreitungszeit im Voraus zu berechnen, wenn das Ultraschallsignal (1203) durch das j-te Ultraschallelement (101, 101A, 101B) empfangen wird, wobei es in einem kleinen Bereich reflektiert wird, Speichern der Ausbreitungszeit im Voraus als eine der physikalischen Größen des jeweiligen kleinen Bereichs und daraufhin Auslesen von Daten aus dem Ultraschallausbreitungsmodell, um die Ausbreitungszeit zu erhalten, die für die Konfiguration des Ultraschallbilds erforderlich ist.

5. Strahlungstherapiesystem, das Folgendes umfasst:

   das System zum Messen einer Körpergewebeposition nach Anspruch 1; und
   eine Strahlungssteuereinheit, die konfiguriert ist, einen Bestrahlungspunkt auf der Basis einer Körpergewebeposition, die unter Verwendung des Systems zum Messen einer Körpergewebeposition gemessen wird, zu steuern.

6. Diagnostisches Bildgebungssystem, das Folgendes umfasst:

   das System zum Messen einer Körpergewebeposition nach Anspruch 1; und
   eine Gate-Steuereinheit (402), die konfiguriert ist, eine Bildgebungszeit auf der Basis einer Körpergewebeposition, die unter Verwendung des Systems zum Messen einer Körpergewebeposition gemessen wird, zu steuern.

**7.** Verfahren zum Messen einer Körpergewebeposition, das die folgenden Schritte umfasst:

einen Schritt zum Messen einer Position von mehreren Ultraschallelementen (101, 101A, 101B), die an verschiedenen Abschnitten auf der Oberfläche eines Körpers angeordnet sind, mittels eines äußeren Sensors (103);

einen Schritt zum Berechnen eines Ausbreitungswegs und einer Zeit einer Ultraschallwelle von einem Ultraschallelement zu einer Position eines weiteren Ultraschallelements mittels eines Ultraschall-Ausbreitungsmodells;

einen Schritt zum Konfigurieren eines Ultraschallbilds aus der Ultraschall-Ausbreitungszeit und aus einem Ultraschallsignal, das durch das jeweilige Element empfangen wird;

einen Schritt zum Berechnen einer Position des Ziel-Körpergewebes aus dem Ultraschallbild; und

einen Schritt zum Verstärken und Verarbeiten von elektrischen Signalen, die von den Ultraschallelementen (101) empfangen werden, und zum Senden der elektrischen Signale an die Einheit (104) zum Konfigurieren eines Ultraschallbilds mittels einer Einheit (102) zum Senden und Empfangen von Ultraschall;

wobei die Ultraschallelemente unabhängig vorgesehen sind, so dass sich ihre relative Position in Übereinstimmung mit einer Bewegung ändert.

**8.** Verfahren zum Messen einer Körpergewebeposition nach Anspruch 7, das ferner einen Schritt zum Aktualisieren der Berechnung eines Ultraschall-Ausbreitungsmodells und einer Körpergewebeposition in Übereinstimmung mit einer Änderung der Kontur eines Patienten umfasst.

**9.** Verfahren zum Messen einer Körpergewebeposition nach Anspruch 7, das ferner die folgenden Schritte umfasst:

einen Schritt zum Erzeugen eines Ultraschallmodells; und

einen Schritt zum Speichern in einer Ultraschallmodell-Speichereinheit (105).

**10.** Verfahren zum Messen einer Körpergewebeposition nach Anspruch 9, wobei der Schritt zum Erzeugen eines Ultraschallmodells die folgenden Schritte umfasst:

einen Schritt zum Sammeln von CT-Bildern für jede Atmungsphase;

einen Schritt zum Sammeln von tatsächlichen Ultraschallsignalen in der gleichen Atmungsphase wie das CT-Bild mittels eines Ultraschallelements;

einen Schritt zum Messen einer Position des Ultraschallelements in der gleichen Atmungsphase wie das CT-Bild mittels eines äußeren Sensors (103);

einen Schritt zum Erzeugen eines Anfangsmodells, das ein Ultraschallelement und einen Ziel-Behandlungsabschnitt auf der Basis des CT-Bilds umfasst;

einen Schritt zum Teilen des Anfangsmodells in kleine Bereiche und zum Einstellen einer Dichte und einer Schallgeschwindigkeit in jedem kleinen Bereich;

einen Schritt zum Berechnen eines virtuellen Ultraschallsignals auf der Basis des Anfangsmodells, der Ultraschallelement-Position und der Dichte und der Schallgeschwindigkeit; und

einen Schritt zum Vergleichen des tatsächlichen Ultraschallsignals (1203) mit dem virtuellen Ultraschallschallsignal, um die Dichte und die Schallgeschwindigkeit des kleinen Bereichs zu aktualisieren.

**11.** Verfahren zum Messen einer Körpergewebeposition nach Anspruch 10,
wobei der Schritt zum Einstellen der Dichte und die Schallgeschwindigkeit in dem jeweiligen kleinen Bereich die Dichte und die Schallgeschwindigkeit auf der Basis der Intensität des CT-Bilds einstellt.


**Revendications**

**1.** Système de mesure de localisation de tissu corporel comprenant :

une pluralité d'éléments à ultrasons (101, 101A, 100B) placés dans différentes parties sur la surface d'un corps ;

un capteur externe (100) configuré pour mesurer une position des éléments à ultrasons ;

une unité de maintien de modèle de propagation des ultrasons (105) configurée pour maintenir un modèle de propagation des ultrasons ;

une unité de calcul de temps de propagation des ultrasons (106) configurée pour calculer un temps de propagation des ultrasons sur la base du modèle d'ultrasons et de la position des éléments à ultrasons ;

une unité de configuration d'image à ultrasons (104) configurée pour configurer une image à ultrasons à partir du temps de propagation des ultrasons également ainsi qu'un signal à ultrasons reçu par chaque élément ;

une unité de calcul de localisation de tissu corporel (107) configurée pour calculer une localisation d'un tissu corporel cible à partir de l'image à ultrasons,

une unité d'émission/réception des ultrasons (102) configurée pour amplifier et traiter des signaux électriques reçus depuis les éléments à ultrasons (101), et pour émettre les signaux électriques vers l'unité de configuration d'image à ultrasons (104),

dans lequel les éléments à ultrasons (101) sont prévus de manière indépendante, de sorte que leur position relative varie en accord avec un déplacement.

2. Système de mesure de localisation de tissu corporel selon la revendication 1, comprenant en outre :
un dispositif d'affichage configuré pour rendre visible l'image à ultrasons configurée par l'unité de configuration d'image à ultrasons également ainsi que localisation de tissu corporel cible calculée par l'unité de calcul de localisation de tissu corporel.

3. Système de mesure de localisation de tissu corporel selon la revendication 1, comprenant en outre :

un processeur d'image TDM (202) ;
une unité de génération de modèle d'ultrasons (203) configurée pour générer un modèle d'ultrasons sur la base d'images collectées par le processeur d'image TDM, du modèle d'ultrasons et de la position d'éléments à ultrasons ; et
un moyen de maintien configuré pour stocker le modèle d'ultrasons dans l'unité de maintien de modèle de propagation des ultrasons.

4. Système de mesure de localisation de tissu corporel selon la revendication 1, dans lequel :
un i-ième élément à ultrasons (101, 101A, 101B) dans l'unité de génération de modèle d'ultrasons est configuré :
pour émettre un signal à ultrasons en avance, pour calculer le temps de propagation en avance quand le signal à ultrasons (1203) est reçu par un j-ième élément à ultrasons (101, 101 A, 101 B) tout en étant réfléchi à l'intérieur d'une petite région, pour maintenir le temps de propagation en avance à titre d'une des quantités physiques de la petite région respective, et ensuite pour lire des données depuis le modèle de propagation d'ultrasons pour obtenir le temps de propagation requis pour la configuration d'images à ultrasons.

5. Système de thérapie par radiation comprenant :

le système de mesure de localisation de tissu corporel selon la revendication 1 ; et
une unité de commande de radiation configurée pour commander un point d'irradiation de radiation sur la base d'une localisation de tissu corporel mesurée en utilisant le système de mesure de localisation de tissu corporel.

6. Système d'imagerie de diagnostic comprenant :

le système de mesure de localisation de tissu corporel selon la revendication 1 ; et
une unité de commande à passerelle (402) configurée pour commander une temporisation d'imagerie sur la base d'une localisation de tissu corporel mesurée en utilisant le système de mesure de localisation de tissu corporel.

7. Procédé de mesure de localisation de tissu corporel comprenant :

une étape consistant à mesurer une position d'une pluralité d'éléments à ultrasons (101, 101A, 101B), qui sont placés dans différentes parties sur la surface d'un corps, au moyen d'un capteur externe (103) ;
une étape consistant à calculer un trajet et un temps de propagation d'une onde à ultrasons depuis un élément à ultrasons jusqu'à une position d'un autre élément à ultrasons au moyen d'un modèle de propagation d'ultrasons ;
une étape consistant à configurer une image à ultrasons à partir du temps de propagation des ultrasons et d'un signal à ultrasons reçu par chaque élément ;
une étape consistant à calculer une localisation du tissu corporel cible à partir de l'image à ultrasons ; et
une étape consistant à amplifier et à traiter des signaux électriques reçus depuis les éléments à ultrasons (101) et à émettre les signaux électriques vers l'unité de configuration d'image à ultrasons (104) au moyen d'une unité d'émission/réception d'ultrasons (102) ;
dans lequel les éléments à ultrasons sont prévus de manière indépendante, de sorte que leur position relative varie en accord avec un déplacement.

8. Procédé de mesure de localisation de tissu corporel selon la revendication 7, comprenant en outre une étape consistant à mettre à jour le calcul d'un modèle de propagation des ultrasons et une localisation de tissu corporel en accord avec un changement dans un contour du patient.

9. Procédé de mesure de localisation de tissu corporel selon la revendication 7, comprenant en outre :

une étape consistant à générer un modèle d'ultrasons ; et
une étape consistant à stocker dans une unité de maintien de modèle d'ultrasons (105).

10. Procédé de mesure de localisation de tissu corporel selon la revendication 9,

dans lequel l'étape consistant à générer un modèle d'ultrasons inclut :

une opération consistant à collecter des images TDM pour chaque phase respiratoire ;
une opération consistant à collecter des signaux à ultrasons réels dans la même phase respiratoire que l'image TDM au moyen de l'élément à ultrasons ;
une opération consistant à mesurer une position de l'élément à ultrasons dans la même phase respiratoire que l'image TDM au moyen d'un capteur externe (103) ;
une opération consistant à générer un modèle initial incluant un élément à ultrasons et une partie cible de traitement sur la base de l'image TDM ;
une opération consistant à diviser le modèle initial en petites régions et à établir une densité et une vitesse acoustique dans chaque petite région ;
une opération consistant à calculer un signal à ultrasons virtuel sur la base du modèle initial, de la position de l'élément à ultrasons, et de la densité et de la vitesse acoustique ; et
une opération consistant à comparer le signal à ultrasons réel (1203) au signal à ultrasons virtuels pour mettre à jour la densité et la vitesse acoustique de la petite région.

11. Procédé de mesure de localisation de tissu corporel selon la revendication 10,
dans lequel l'étape consistant à établir la densité et la vitesse acoustique dans chaque petite région établit la densité et la vitesse acoustique sur la base de l'intensité de l'image TDM.

# FIG. 1

# FIG. 2

# FIG. 3

# FIG. 4

# FIG. 5

S101 — START

↓

S102 — COLLECT CT IMAGE

↓

S103 — COLLECT ACTUAL ULTRASONIC SIGNAL

↓

S104 — MEASURE ULTRASONIC ELEMENT POSITION BY EXTERNAL SENSOR

↓

S105 — GENERATE INITIAL ULTRASONIC PROPAGATION MODEL

↓

S106 — CALCULATE VIRTUAL ULTRASONIC SIGNAL

↓

S107 — MATCH WITH SUFFICIENT ACCURACY?

NO → S108 — UPDATE MODEL

YES ↓

S109 — HOLD MODEL

↓

S110 — END

# FIG. 6

1201

1202

1203

$\Phi_{11}(t)$    t    $\Phi_{21}(t)$    t

$\Phi_{12}(t)$    $\Phi_{22}(t)$

$\Phi_{12}(t)$    $\Phi_{23}(t)$    ...

$\Phi_{14}(t)$    $\Phi_{24}(t)$

FIG. 7

1201B

1201A

1202

# FIG. 8

S201 — START

S202 — COLLECT ACTUAL ULTRASONIC SIGNAL

S203 — MEASURE ULTRASONIC ELEMENT POSITION BY EXTERNAL SENSOR

S204 — SELECT MODEL

S205 — CALCULATE PROPAGATION TIME BETWEEN ULTRASONIC ELEMEN

S206 — INTEGRATE SIGNAL OF MODEL SMALL REGION

S207 — IMAGE

S208 — END

# FIG. 9

S301 — ( START )

S302 — CALCULATE RELATIVE COORDINATES OF EXTERNAL SENSOR AND ULTRASONIC ELEMENT

S303 — CALCULATE RELATIVE COORDINATES OF ULTRASONIC ELEMENT AND TARGET TISSUE

S304 — CALCULATE ABSOLUTE COORDINATE OF TARGET TISSUE

S305 — ( END )

# FIG. 10

# FIG. 11

FIG. 12

**EP 3 363 367 B1**

## REFERENCES CITED IN THE DESCRIPTION

**Patent documents cited in the description**

- JP 2003117010 A
- US 2015051480 A1